# EUROPÄISCHE PATENTSCHRIFT

(11) **EP 0 949 251 B1**
(45) Veröffentlichungstag und Bekanntmachung des Hinweises auf die Patenterteilung: **01.04.2009**
(21) Anmeldenummer: 99810280.0
(22) Anmeldetag: 01.04.1999
(51) Int. Cl.: C07D 251/22, C07D 405/12, A61Q 17/04

(54) **Diresorcinyltriazine als Lichtschutzmittel**
Diresorcinyltriazines as sunscreen agents
Diresorcinyltriazines comme agents de protection contre la lumière

(30) Priorität: 09.04.1998 EP 98810314
(43) Veröffentlichungstag der Anmeldung: 13.10.1999
(73) Patentinhaber: Ciba Holding Inc., 4057 Basel (CH)
(72) Erfinder: Ehlis, Thomas, 79106 Freiburg (DE); Borsos, Elek, 4127 Birsfelden (CH); Luther, Helmut, 79639 Grenzach-Wyhlen (DE); Herzog, Bernd, 79639 Grenzach-Wyhlen (DE)

(56) Entgegenhaltungen:
- EP-A- 0 165 608
- EP-A- 0 557 247
- BE-A- 661 225
- US-A- 3 293 247
- CHEMICAL ABSTRACTS, vol. 118, no. 24, 1993 Columbus, Ohio, US; abstract no. 240480e, SUDO ET AL.: "Sunscreens containing (hydroxyphenyl)triazines" Seite 445; XP002075183 & JP 05 025029 A (DAIKYO GOMU SEIKO) 1993
- H. KOOPMAN ET AL.: "Investigations on herbicides II" RECUEIL DES TRAVAUX CHIMIQUES DES PAYS-BAS., Bd. 78, 1959, Seiten 967-980, XP002075181 AMSTERDAM NL
- H. KOOPMAN ET AL.: "U.V. spectra of derivatives of 1,3,5-triazine" RECUEIL DES TRAVAUX CHIMIQUES DES PAYS-BAS., Bd. 80, 1961, Seiten 158-172, XP002075182 AMSTERDAM NL
- BRUNETTI, H. ET AL: "Synthesis of asymmetrically substituted o-hydroxyphenyl-s-triazines" HELV. CHIM. ACTA (1972), 55(5), 1566-95 , XP002106234

## Beschreibung

Die vorliegende Erfindung betrifft neue Diresorcinyl-Alkoxy- und -aryloxy-Triazine, Verfahren zur Herstellung dieser Verbindungen sowie deren Verwendung in kosmetischen Mitteln.

Die neuen Diresorcinyl-Alkoxy- und Aryloxy-Triazine entsprechen der Formel 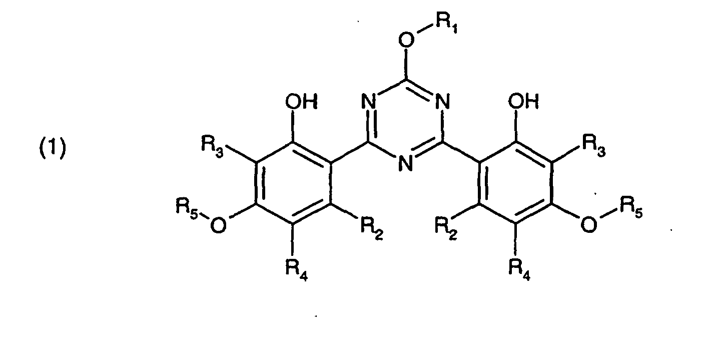 worin
- R₁: 2-Decyl-hexadecyl; isooctadecyl; n-octadecyl; 2-hexyldecyl; 2-ethylhexyl;
- R₂, R₃ und R₄,: unabhängig voneinander Wasserstoff, Hydroxy-, C₁-C₃₀-Alkyl, C₁-C₃₀-Alkenyl,
und
- R₅: Wasserstoff;
bedeuten.

Alkyl bedeutet einen verzweigten oder unverzweigten Kohlenwasserstoffrest wie z.B. Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl,1,1,3,3-Tetramethylbutyl, 1-Methylheptyl, 3-Methylheptyl, n-Octyl, iso-Octyl; 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl oder Octadecyl.

Alkenyl umfasst im Rahmen der angegebenen Bedeutungen u.a. Allyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Dodec-2-enyl, n-Octadec-4-enyl oder 3,7,11,11-Tetramethyl-2,6,10-undecatrienyl.

Als Hintergrund literatur werden die Dokumente US 3293247, BE 661 225, CHEM ABS, vol 118, no 24,1993 & JP 05025029, EP 0165 608, EP 0557247 zitiert.

Weiter Beispielhafte Vertreter der erfindungsgemässen Diresorcinyl-Alkoxy-Triazine sind in der folgenden Tabelle zusammengestellt:

| Tabelle 1: | |
|---|---|
| | 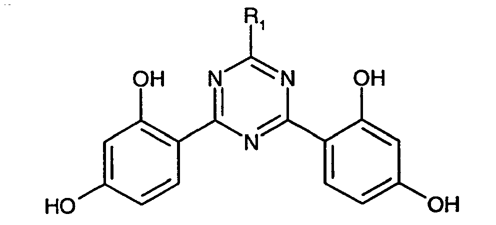 |

| Verbindung der Formel | R₁ |
|---|---|
| (3) | 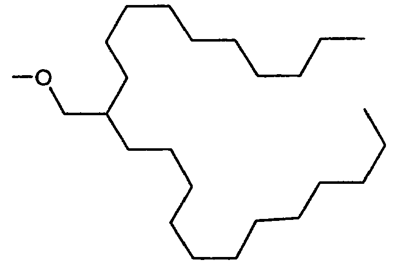 |
| (4) | -O-iso-C₁₈H₃₈ |
| (5) | 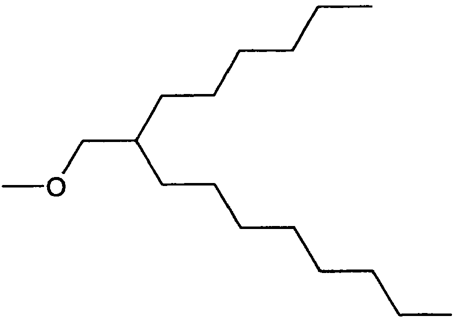 |
| (6) | -O-n-C₁₈H₃₇ |
| (7) | -O-2-Ethyl-hexyl |
| (8)* | 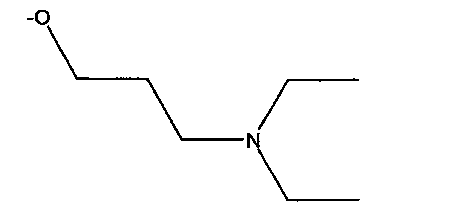 |
| (9)* | 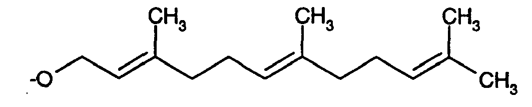 |
| (10)* | 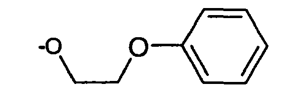 |
| (11)* | 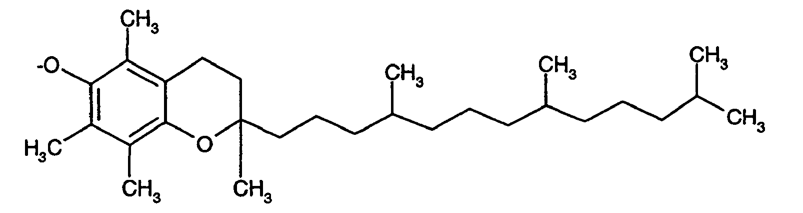 |
| (12)* | 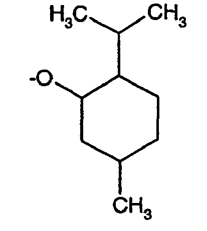 |
| (13)* | 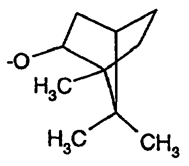 |
| (14)* | 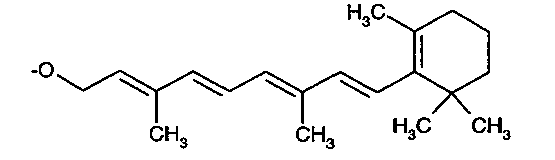 |
| (15)* | 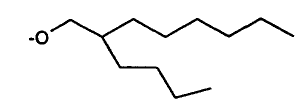 |
| (16)* | 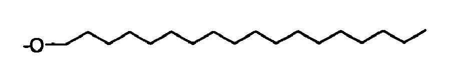 |
| (17)* | 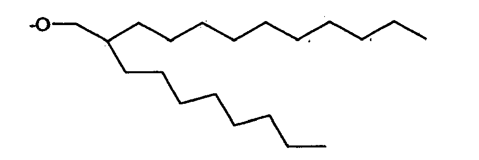 |
| (18)* | 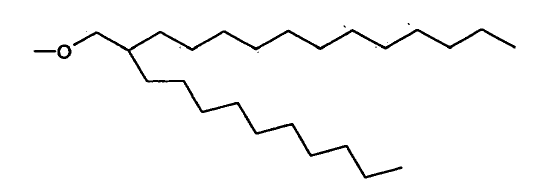 |
| (19)* | 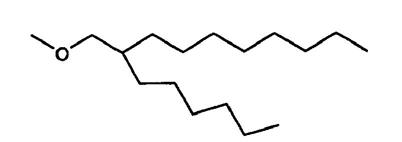 |

| | |
|---|---|
| * Die Beispiele sind nicht Gegenstand der Erfindung | |

Die Herstellung der erfindungsgemässen Diresorcinyl-Akoxy- und -aryloxy-Triazine erfolgt z.B. durch Umsetzung von Cyanurchlorid mit dem Alkohol R₁-OH zu den Aryloxy- bzw.-alkoxy-dichlor-s-triazinen der Formel (2). In einer Friedel-Crafts-Reaktion werden in der zweiten Reaktionsstufe die neuen Verbindungen der Formel (1) erhalten. Die Reaktion lässt sich schematisch folgendermassen darstellen:

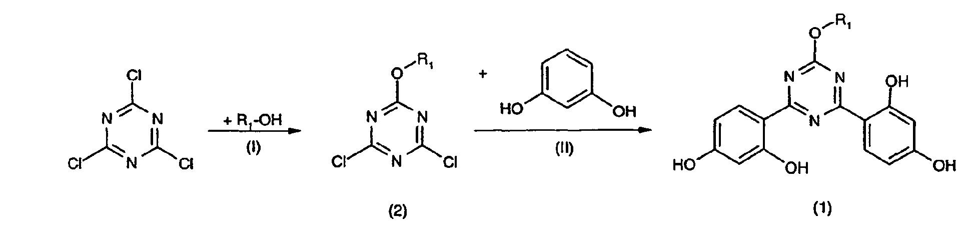

Die erste Reaktionsstufe wird gewöhnlich in Anwesenheit eines Lösungsmittel wie z.B. Aceton, Methylethylketon, Dimethylacetamid, Toluol, oder Xylol durchgeführt.

Die Temperaturen reichen dabei von 0 bis 130, insbesondere 20 bis 70°C; die Reaktionszeiten von 1 bis 48, vorzugsweise von 2 bis 10 Stunden.

In der zweiten Reaktionsstufe werden als Lösungsmittel gewöhnlich Toluol, Nitrotoluol, Nitrobenzol, Anisol, Xylol, Benzol, Sulfolan, Chlorbenzol, Dichlorbenzol, Kohlenwasserstoffe (z.B. lsooctan), chlorierte Kohlenwasserstoffe, Nitroalkane, Schwefelkohlenstoff, Schwefeldioxid und Gemische aus den genannten Lösungsmitteln verwendet.

Die Temperaturen betragen dabei von -10 bis 200, insbesondere 0 bis 100°C.

Die Reaktionszeiten reichen von 1 bis 100, vorzugsweise von 2 bis 50 Stunden.

Die zweite Reaktionsstufe wird gewöhnlich in Anwesenheit eines Katalysators durchgeführt. Als Katalysatoren kommen z.B. in Betracht: Aluminiumchlorid, Aluminiumbromid, Zinnchlorid, Titantetrachlorid, Bortrifluorid sowie andere Lewis-Säuren.

Der Katalysator wird dabei in einer Menge von 0.1 bis 3 Mol pro Mol reaktives Chlor. eingesetzt.

In Friedel-Crafts-Reaktionen mit Dichlor-alkoxy-s-triazinen werden häufig Dealkylierungen im Alkoxyrest beobachtet. Unter erfindungsgemässen Bedingungen, d.h. Steuerung der Temperatur; Menge des Katalysators, Dosierung des Katalysators verläuft die Reaktion besonders glatt und ohne merkliche Dealkylierung.

Eine weitere Synthesemöglichkeit für die erfindungsgemässen Diresorcinyl-Alkoxy- und Aryloxy-Triazine erfolgt durch Umsetzung von Cyanurchlorid mit Resorcin zum 2-Chlor-4,6-resorcinyl-triazin (Verbindung der Formel (20) in der ersten Reaktionsstufe und Umsetzung mit HO-R₁ zur Verbindung der Formel (1) in einer zweiten Reaktionsstufe nach folgendem Schema:

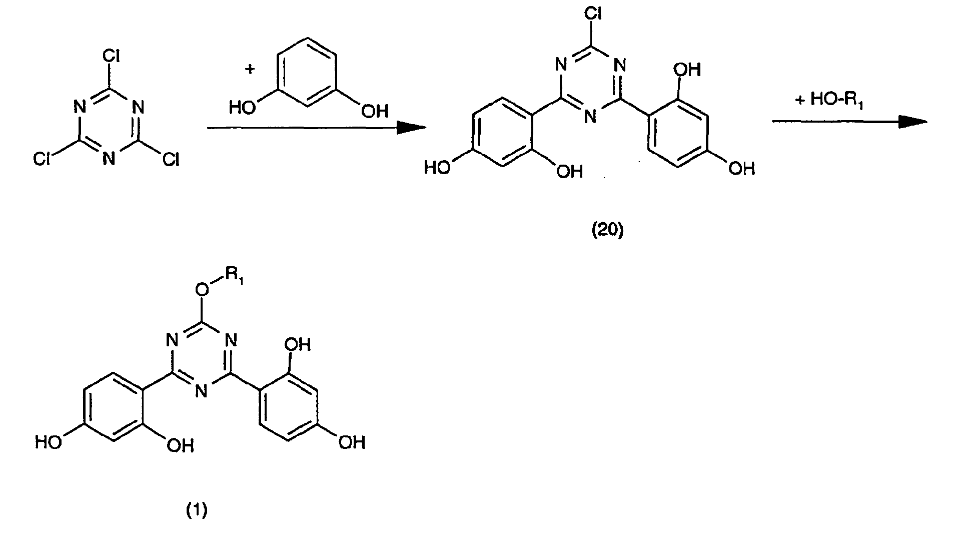

R₁ und R₅ haben dabei die für Formel (1) angegebene Bedeutung.

Die erfindungsgemässen Diresorcinyl-Akoxy- und Aryloxy-Triazine der Formel (1) besitzen ein Absorptionsmaximum von ca. 350 nm, d.h. es handelt sich bei diesen Verbindungen um UV-A-Absorber. Die Verbindungen eignen sich daher insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Sie können sowohl gelöst als auch im mikronisierten Zustand verwendet werden.

Liegen die erfindungsgemässen UV-Absorber in mikronisierter Form vor, so haben sie gewöhnlich eine mittlere Partikelgrösse von 0,02 bis 2, vorzugsweise 0,05 bis 1,5, und ganz besonders von 0,1 bis 1,0 µm. Die Methoden zur Mikronisierung sind z.B. in der GB A-2303549 beschrieben.

Als Mahlapparate zur Herstellung der erfindungsgemässen mikronisierten organischen UV-Absorber können z.B. eine Düsen-, Kugel-, Vibrations- oder Hammermühle, vorzugsweise eine Hochgeschwindigkeits-Rührmühle verwendet werden. Die Mahlung erfolgt vorzugsweise mit einer Mahlhilfe, wie z.B. einem alkylierten Vinylpyrrolidon-Polymer, einem Vinylpyrrolidon-Vinylacetat-Copolymer, einem Acylglutamat oder insbesondere einem Phospholipid.

Auf Grund ihrer Lipophilität lassen sich die Verbindungen (1) besonders dann gut in Öl- und fetthaltige kosmetische Formulierungen einarbeiten, wenn R₁ ein verzweigter Alkylrest mit mehr als 8 C-Atomen ist.

Einen weiteren Erfindungsgegenstand bildet ein kosmetisches Präparat, enthaltend mindestens eine Verbindung der Formel (1), sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können, wie weiter oben erwähnt, durch übliche Mahlmethoden, auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe durchgeführt.

Das kosmetische Präparat kann neben dem erfindungsgemässen UV-Absorber auch noch einen oder mehrere weitere UV-Schutzstoffe der folgenden Substanzklassen enthalten:
1. p-Aminobenzoesäurederivate, wie z.B. 4-Dimethylaminobenzoesäure-2-ethylhexylester;
2. Salicylsäurederivate, wie z.B. Salicylsäure-2-ethylhexylester;
3. Benzophenonderivate, wie z.B. 2-Hydroxy-4-methoxybenzophenon und sein 5-sulfonsäurederivat;
4. Dibenzoylmethanderivate, wie z.B. 1 -(4-tert.-Butylphenyl)-3-(4-methoxyphenyl)propan-1,3-dion;
5. Diphenylacrylate, wie z.B. 2-Ethylhexyl-2-cyano-3,3-diphenyl acrylat und 3-(Benzofuranyl)-2-cyanoacrylat;
6. 3-lmidazol-4-yl-acrylsäure und -ester;
7. Benzofuranderivate, insbesondere 2-(p-Aminophenyl)benzofuranderivate, beschrieben in der EP-A-582,189, US-A-5,338,539, US-A-5,518,713 und der EP-A-613,893;
8. polymere UV-Absorber wie z.B. die in der EP-A-709,080 beschriebenen Benzylidenmalonatderivate;
9. Zimtsäurederivate, wie z.B. die in der US-A-5,601,811 und WO 97/00851 offenbarten 4-Methoxyzimtsäure-2-ethylhexylester bzw. lsoamylester oder Zimtsäurederivate;
10. Campherderivate, wie z.B. 3-(4'-Methyl)benzyliden-bornan-2-on, 3-Benzyliden-bornan-2-on, N-[2(und 4)-2-Oxyborn-3-yliden-methyl)-benzyl]acrylamid-Polymer, 3-(4'-Trimethylammonium)-benzyliden-bornan-2-on methylsulfat, 3,3'-(1,4-Phenylendimethin)-bis(7,7-dimethyl-2-oxo-bicyclo-[2.2.1]heptan-1-methansulphonsäure) und Salze, 3-(4'-Sulfo)-benzyliden-bornan-2-on und Salze;
11. Trianilino-s-Triazinderivate, wie z.B. 2,4,6-Trianilin-(p-carbo-2'-ethyl-1'-oxi)-1,3,5-triazin sowie die in der US-A-5,332,568, EP-A-517,104, EP-A-507,691, WO 93/17002 und EP-A-570,838 offenbarten UV-Absorber;
12. 2-Hydroxyphenyl-Benzotriazol-Derivate;
13. 2-Phenylbenzimidazol-5-sulfonsäure und deren Salze;
14. Menthyl-o-aminobenzoat.
15. TiO₂ (unterschiedlich umhüllt), ZnO und Mica.

Auch die in "Sunscreens", Eds. N.J. Lowe, N.A.Shaath, Marcel Dekker, Inc., New York and Basel oder in Cosmetics & Toiletries (107), 50ff (1992) beschriebenen UV-Absorber können als zusätzliche UV-Schutzstoffe in der erfindungsgemässen Formulierung verwendet werden.

Weiterhin kann das erfindungsgemässe kosmetische Präparat auch zusammen mit bekannten Antioxidantien, wie z.B. Vitamin E, Carotinoiden oder HALS (="Hindered Amine Light Stabilizers")-Verbindungen eingesetzt werden.

Das erfindungsgemässe kosmetische Präparat enthält 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung des kosmetischen Präparats kann durch physikalisches Mischen des oder der UV-Absorber mit dem Hilfsstoff durch gewöhnliche Methoden, wie z.B. durch einfaches Zusammenrühren der Einzelkomponenten erfolgen.

Das erfindungsgemässe kosmetische Präparat kann als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Für das erfindungsgemässe kosmetische Präparat kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Das kosmetische Präparat kann auch weitere Komponenten, wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft-und Farbstoffe enthalten.

Das erfindungsgemässe kosmetische Präparat zeichnet sich durch exzellenten Schutz der menschlichen Haut gegen den schädigenden Einfluss von Sonnenlicht aus.

Die erfindungsgemässen Diresorcinyl-alkoxy- und -aryloxy-s-Triazine zeichnen sich durch eine grosse thermische Stabilität aus und finden daher Verwendung als Stabilisatoren für organische Polymere, insbesondere Lacke, gegen deren Schädigung durch Licht, Sauerstoff und Wärme.

Das mit den erfindungsgemässen Verbindungen stabilisierte Material zeichnet sich durch hervorragende Beständigkeit gegen Einflüsse durch Witterung und Licht, sowie hervorragende Photostabilität des eingearbeiteten Stabilisators aus.

Die zu stabilisierenden Materialien können z.B. Öle, Fette, Wachse oder Biocide sein. Von besonderem Interesse ist die Verwendung in polymeren Materialien, wie sie in Kunststoffen, Kautschuken, Anstrichstoffen, Lacken, fotografischem Material oder Klebstoffen vorliegen.

Gegenstand der Erfindung ist daher auch eine Zusammensetzung enthaltend
(A) ein gegen Schädigung durch Licht, Sauerstoff und/oder Hitze empfindliches organisches Material und
(B) als Stabilisator eine Verbindung der Formel (1)

Die Erfindung betrifft auch ein Verfahren zum Stabilisieren von organischem Material gegen Schädigung durch Licht, Sauerstoff und/oder Hitze, das dadurch gekennzeichnet ist, dass man diesem als Stabilisator eine Verbindung der Formel (1) zusetzt, sowie die Verwendung der Verbindung der Formel (1) zum Stabilisieren von organischem Material.

Die Menge des zu verwendenden Stabilisators richtet sich nach dem zu stabilisierenden organischen Material und der beabsichtigten Verwendung des stabilisierten Materials. Im allgemeinen enthält die erfindungsgemässe Zusammensetzung auf 100 Gew.-Teile der Komponente (A) 0,01 bis 15, insbesondere 0,05 bis 10, und vor allem 0,1 bis 5 Gew.-Teile des Stabilisators (Komponente(B)).

Der Stabilisator (Komponente (B)) kann auch ein Gemisch sein von mehreren Verbindungen der Formel (1). Die erfindungsgemässen Zusammensetzungen können ausser den erfindungsgemässen Verbindungen noch andere Stabilisatoren oder sonstige Zusätze enthalten, wie z.B. Antioxidantien, weitere Lichtschutzmittel, Metalldesaktivatoren, Phosphite oder Phosphonite.

Die Art und Menge der zugesetzten weiteren Stabilisatoren wird von der Art des zu stabilisierenden Substrates und dessen Verwendungszweck bestimmt; häufig werden 0,1 bis 5 Gew.-%, bezogen auf das zu stabilisierende Polymer, verwendet.

Die Einarbeitung in die organischen Polymere, beispielsweise in die synthetischen organischen, insbesondere thermoplastischen Polymere, kann durch Zusatz der erfindungsgemässen Triazinverbindungen und gegebenenfalls weiterer Additive nach den in der Technik üblichen Methoden erfolgen. Die Einarbeitung kann zweckmässig vor oder während der Formgebung, beispielsweise durch Mischen der pulverförmigen Komponenten oder durch Zusatz des Stabilisators zur Schmelze oder Lösung des Polymeren, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Gemische in Polymere besteht in deren Zugabe vor oder während der Polymerisation der entsprechenden Monomeren bzw. vor der Vernetzung.

Die so erhaltenen stabilisierten Polymerzusammensetzungen können nach den üblichen Methoden, wie z.B. durch Heisspressen, Spinnen, Extrudieren oder Spritzgiessen, in geformte Gegenstände überführt werden, wie z.B. in Fasern, Folien, Bändchen, Platten, Stegplatten, Gefässe, Rohre und sonstige Profile.

Die so stabilisierten Polymere zeichnen sich aus durch hohe Witterungsbeständigkeit, vor allem durch hohe Beständigkeit gegen UV-Licht. Sie behalten dadurch auch im Aussengebrauch lange Zeit ihre mechanischen Eigenschaften sowie ihre Farbe und ihren Glanz.

In den folgenden Beispielen beziehen sich die Prozentsätze auf das Gewicht. Die Mengen beziehen sich bei den eingesetzten Dis-Resorcinyl-Triazinverbindungen auf die Reinsubstanz.

Herstellundsbeispiele

### * Beispiel 1a: Herstellung der Verbindung der Formel (Mit "*" gekennzeichnete Verbindungen sind nicht Gegenstand der Erfindung)

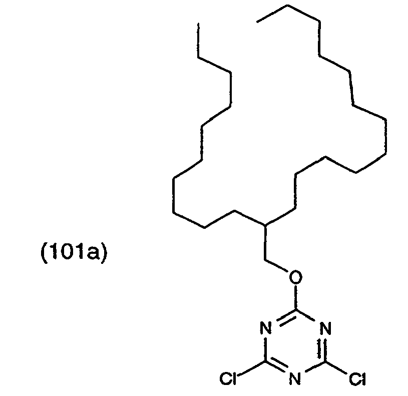

Cyanurchlorid (9,22 g , 0,05 mol) wird in Toluol (80ml) vorgelegt. Innerhalb von 40 min wird ein Gemisch aus 2-Decyl-1-tetradecanol (20,1 g ,0.057 mol), Dimethylacetamid (6,53 g ,0.075 mol) und Toluol (20ml) bei 30-55 °C zugetropft, 4h bei 50°C nachgerührt und über Kieselgur abfiltriert.

Das Filtrat wird mit tert.-Butyl-methyl-keton (50ml) und eiskalter 10%iger NaCl-Lösung (150ml) versetzt und ausgeschüttelt. Die organische Phase wird nochmals mit 10%iger NaCl-Lösung gewaschen, abgetrennt und über Na₂SO₄ getrocknet. Nach Abziehen des Lösungsmittels wird das erhaltene Öl säulenchromatographisch aufgearbeitet (Kieselgel, Toluol/Hexan 7:3).

Man erhält ein farbloses Öl der Verbindung der Formel (101a).
Ausbeute: 18,4 g (73 %)
¹³C NMR (90 MHz, CDCl₃, TMS): δ = 14,50; 23,09; 27,08; 29,74; 29,76; 29,97; 30,04; 30,26; 30,32; 31,31; 32,32; 37,75; 73,68; 171,63; 172,84.

| Elementaranalyse: | | | |
|---|---|---|---|
| | %C | %H | %N |
| Berechnet: | 64,52 | 9,83 | 8,36 |
| Gefunden: | 65,0 | 9,9 | 8,2 |

### * Beispiel 1b: Herstellung der Verbindung der Formel (101)

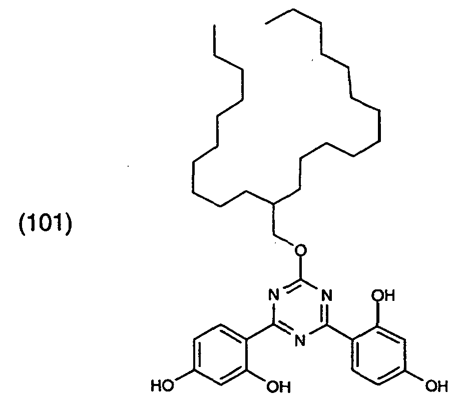

Resorcin (4,9 g, 0.045 mol) wird in Nitrobenzol (40ml) vorgelegt. Bei 10-15°C wird gepulvertes Aluminiumchlorid (5,9 g, 0.044 mol) portionsweise eingetragen. 30min wird nachgerührt, die Verbindung der Formel (101 a) (10.05 g, 0.020 mol) gelöst in Nitrobenzol (10ml), innerhalb von 30min bei 10-15°C zugetropft und 5 Stunden nachgerührt. Bei Raumtemperatur wird weitere 3 Stunden nachgerührt. Die Reaktion kann im Dünnschichtchromatogramm verfolgt werden (Kieselgel, Toluol/Aceton 9:1).

Das Reaktionsgemisch wird unter Rühren in eine Mischung aus Eiswasser (100 ml) und 2N-HCl (25 ml) gegossen. Dabei scheidet sich ein Niederschlag ab. Nitrobenzol-Reste werden mittels Wasserdampfdestillation entfernt. Der feste Rückstand wird in Aceton gelöst, mit Na₂SO₄ getrocknet, eingeengt und säulenchromatographisch getrennt (Kieselgel, Toluol/Essigester 8:2). Man erhält 5,7g eines gelben Pulvers, welches nach Umkristallisieren aus Hexan/Dioxan 6:4 analysenrein anfällt.

Man erhält hellgelbe Kristalle der Verbindung der Formel (101).
Ausbeute: 4,1 g (31 %)
Schmp.: 165-166 °C
UV/Vis (EtOH): λₘₐₓ (ε) = 350 (34482) nm
¹³C NMR (90MHz, D₆-DMSO, TMS): δ = 14,6 (CH₃); 23,0 (CH₂); 27,0 (CH₂); 29,8 (CH₂); 30,0 (CH₂); 30,06 (CH₂); 30,11 (CH₂); 30,3 (CH₂); 31,3 (CH₂); 32,3 (CH₂); 37,6 (CH); 71,2(CH₂O); 103,8 (CH); 109,0 (CH); 109,4 (CH); 131,6 (CH); 164,6 (C_{q}); 165,1 (C_{q}); 167,8 (C_{q}); 171,5 (C_{q}).

| Elementaranalyse: | | | |
|---|---|---|---|
| | %C | %H | %N |
| Berechnet: | 72,08 | 9,15 | 6,47 |
| Gefunden: | 72,04 | 9,16 | 6,48 |

### Beispiel 2a: Herstellung der Verbindung der Formel (102a):

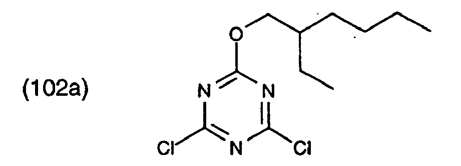

Die Verbindung der Formel (102a) wird analog der Verbindung (101a) aus Beispiel 1 hergestellt. Als Alkoholkomponente wird 2-Ethyl-hexanol verwendet.
Das flüssig anfallende Rohprodukt wird durch Destillation im Hochvakuum (Sdp. 118-119° C/0.2 torr) gereinigt.

Die Reinigung des Rohprodukts kann auch säulenchromatographisch an Kieselgel (Toluol/Hexan 9:1) erfolgen.

Die Verbindung wurde bereits in US-A-3,542,752 (American Cyanamid) beschrieben.

### Beispiel 2b: Herstellung der Verbindung der Formel (102):

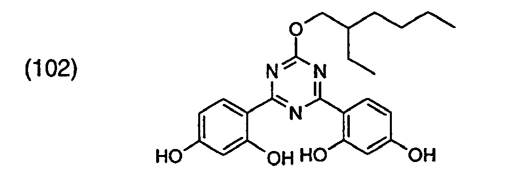

Die Verbindung der Formel (102a) (11,1 g, 0,040 mol), Resorcin (9,7 g, 0,088 mol), Xylol (80 ml) und Sulfolan (30 ml) werden vorgelegt. Bei 35-40°C wird innerhalb von 20 min pulverisiertes Aluminiumchlorid (11,7 g, 0,088 mol) eingetragen und 5 Stunden nachgerührt. Das zweiphasige Reaktionsgemisch wird getrennt. Die untere orangerote Phase lässt man in eine Mischung aus Eiswasser (250 ml) und 32%iger Salzsäure (20 ml) einlaufen. Der ausgeschiedene Feststoff wird abfiltriert, mit Aceton gewaschen und getrocknet. Aus Dioxan wird mehrfach umkristallisiert.

Man erhält hellgelbe Kristalle der Verbindung der Formel (102).

| | |
|---|---|
| Löslichkeit in Ethanol (25°C): | 1,50% |
| Smp. | 235-236 °C |
| Ausbeute: | 5 g (29 %) |
| UV/Vis (EtOH): | λₘₐₓ (ε) = 350 (39177) nm |
| ¹³C NMR (90MHz, D₆-DMSO, TMS): | δ= 11,6 (CH₃); 14,7 (CH₃); 23,3 (CH₂); 24,0 (CH₂); 29,3 (CH₂); 30,6 (CH₂); 38,9 (CH); 71,0 (CH₂O); 103,9 (CH); 109,5 (CH); 131,9 (CH); 164,4 (C_{q}); 165,1 (C_{q}); 167,9 (C_{q}); 171,5 (C_{q}). |

| Elementaranalyse: | | | |
|---|---|---|---|
| | %C | %H | %N |
| Berechnet: | 64.93 | 6.40 | 9.88 |
| Gefunden: | 64.6 | 6.4 | 9.9 |

### Beispiel 3a: Herstellung der Verbindung der Formel (103a)

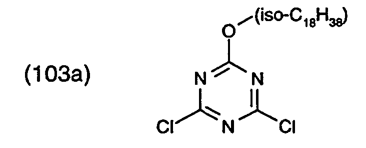

Die Verbindung der Formel (103a) wird analog der Verbindung (101a) aus Beispiel 1 hergestellt. Als Alkoholkomponente wird Isooctadecanol-Isomerengemisch (CA-Reg.No. 27458-93-1) verwendet.

### Beispiel 3b: Herstellung der Verbindung der Formel (103):

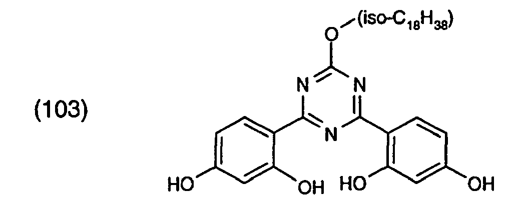

Resorcin (6,6 g, 0.060 mol), Nitrobenzol (40 ml) und Xylol-lsomerengemisch (20 ml) werden vorgelegt. Bei 45-50°C wird gepulvertes Aluminiumchlorid (7,0 g, 0,052 mol) eingetragen und 30 min gerührt. Danach wird bei 0-5°C innerhalb von 1,5 Stunden eine Mischung aus der Verbindung der Formel (103a) (10,5 g, 0,025 mol) und Xylol (10 ml) zugetropft und bei 2-3°C 5 Stunden nachgerührt.

Zur Aufarbeitung lässt man das Reaktionsgemisch in eine Mischung aus Eiswasser (100 ml) und 2N HCl (25 ml) einlaufen. Nitrobenzol wird mittels Wasserdampfdestillation entfernt. Mit Butylmethylether (200 ml) wird das Rohprodukt aus dem Rückstand extrahiert. Die organische Phase wird mit 5%iger NaCl-Lösung gewaschen, getrocknet und vom Lösungsmittel befreit. Säulenchromatographisch (Kieselgel, Toluol/Aceton 9:1) wird das Gemisch anschliessend aufgetrennt.

Man erhält beigefarbene Kristalle der Verbindung der Formel (103).
Ausbeute: 4,6 g (32 %)
Smp.: 166-167°C
UV/Vis (EtOH): λₘₐₓ = 351 nm
¹³C NMR (90MHz, D₆-DMSO, TMS): δ = 13,9 (CH₃); 22,1 (CH₂); 26,1 (CH₂); 28,77 (CH₂); 28,8 (CH₂); 29,0 (CH₂); 29,1 (CH₂); 29,4 (CH₂); 30,6 (CH₂); 31,3 (CH₂); 31,4 (CH₂); 36,7 (CH); 70,5 (CH₂O);103,1 (CH); 108,4 (CH); 108,7 (CH); 131,0 (CH); 163,4 (C_{q}); 164,4 (C_{q}); 167,2 (C_{q}); 170,8 (C_{q}).

| Elementaranalyse: | | | |
|---|---|---|---|
| | %C | %H | %N |
| Berechnet: | 70.06 | 8.37 | 7.43 |
| Gefunden: | 70.2 | 8.5 | 7.3 |

### Beispiel 4a: Herstellung der Verbindung der Formel (104a):

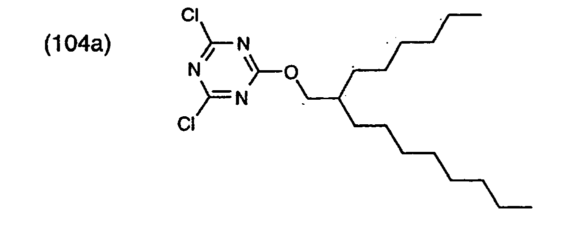

Die Verbindung der Formel (104a) wird analog der Verbindung der Formel (101a) aus Beispiel 1 hergestellt. Als Alkoholkomponente wird 2-Hexyl-decanol verwendet.

### Beispiel 4b: Herstellung der Verbindung der Formel (104)

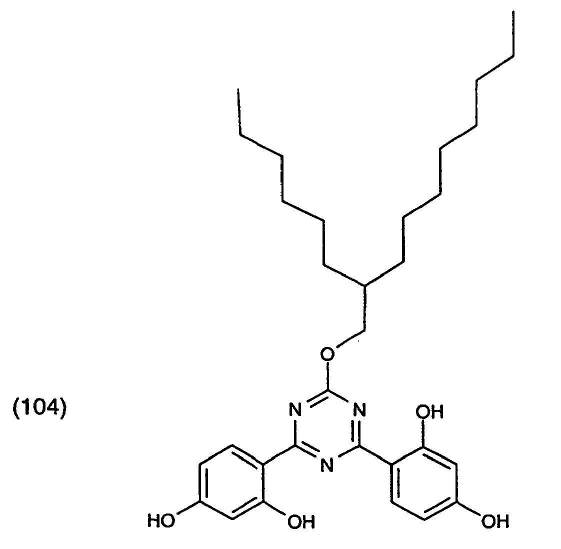

In eine Lösung der Verbindung der Formel (104a) (9,76 g, 0,025 mol) in Toluol (80 ml) wird Resorcin (6,6 g, 0,060 mol) bei 0-5°C innerhalb von 30 min eingetragen. Pulverisiertes Aluminiumchlorid (7,0 g, 0,025 mol) wird anschliessend bei 2°C innerhalb von 30 min in kleinen Portionen in das Reaktionsgemisch eingebracht und 4 Stunden nachgerührt. Das Kältebad wird entfernt und über Nacht bei Raumtemperatur nachgerührt. Die Reaktion kann im Dünnschichtchromatogramm verfolgt werden (Kieselgel, Toluol/Aceton 9:1).

Nach Eingiessen des Reaktionsgemisches in verdünnte Salzsäure (150 ml H₂O + 25ml conc. HCl) erfolgt bei 50°C eine Phasentrennung. Aus der oberen organische Phase wird mittels Wasserdampfdestillation das Toluol entfernt. Der abgeschiedene Feststoff wird mit tert.-Butyl-methylether extrahiert. Das über Na₂SO₄ getrocknete Extrakt wird eingeengt und säulenchromatographisch getrennt (Kieselgel, Toluol/Aceton 8:2).

Man erhält hellgelbe Kristalle der Verbindung der Formel (104) (aus Dioxan/Hexan).
Ausbeute: 10,1 g (74,8 %)
Smp.: 175-176°C
UV/Vis (EtOH.): λₘₐₓ (ε) = 351 (36148) nm
¹³C NMR (90MHz, D₆-DMSO, TMS): δ = 14,7 (CH₃); 23,0 (CH₂); 26,9 (CH₂); 27,0 (CH₂); 29,6 (CH₂); 29,8 (CH₂); 29,9 (CH₂); 30,2 (CH₂); 31,4 (CH₂); 31,5 (CH₂); 32,1 (CH₂); 32,2 (CH₂); 37,6 (CH); 71,3 (CH₂O); 103,9 (CH); 109,2 (CH); 109,5 (CH); 131,9 (CH); 164,5 (C_{q}); 165,1 (C_{q}); 168,0 (C_{q}); 171,6 (C_{q})

| Elementaranalyse: | | | |
|---|---|---|---|
| | %C | %H | %N |
| Berechnet: | 69,25 | 8,06 | 7,81 |
| Gefunden: | 69,1 | 7,9 | 7,.8 |

### * Beispiel 5a: Herstellung der Verbindung der Formel (105a)

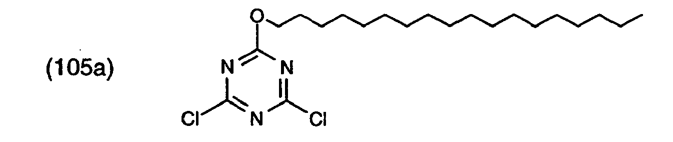

Die Verbindung der Formel (105a) wird analog der Verbindung der Formel (101a) hergestellt. Als Alkoholkomponente wird 1-Octadecanol verwendet.

### * Beispiel 5b: Herstellung der Verbindung der Formel (105):

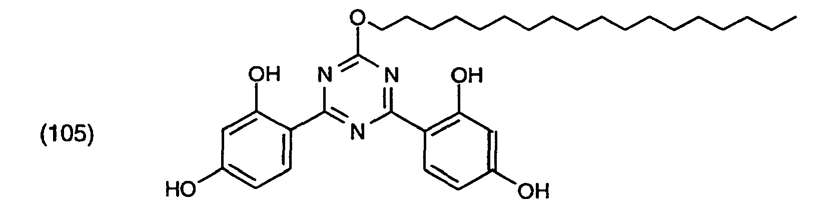

Nitrobenzol (40 ml), Resorcin (6,6 g, 0,06 mol) und Xylol Isomerengemisch (20 ml) werden bei Raumtemperatur vorgelegt. Bei 45-50°C wird pulverisiertes Aluminiumchlorid (7,0 g, 0,05 mol) eingetragen und 30 min. bei 45-50°C nachgerührt. Danach wird bei 0-5°C eine Lösung der Verbindung der Formel (105a) (10,5 g, 0,025 mol) in Xylol-lsomerengemisch (15 ml) innerhalb von 35 min. zugetropft. Es wird 3 Stunden bei 0-5°C und anschliessend 1,5 Stunden bei 5-10°C nachgerührt. Die Reaktionssuspension wird in eine Mischung aus Eiswasser (200 ml) und 4 N Salzsäure (25 ml) eingerührt und auf 50°C erwärmt. Dann wird mit tert. Butyl-methylether (200 ml) extrahiert und die organische Phase am Rotationsverdampfer eingeengt. Der Rückstand wird mit Wasser versetzt und Reste des Nitrobenzol/Xylol-lsomerengemisches mittels Wasserdampfdestillation entfernt. Das Rohprodukt wird aus Dioxan/Aceton 6:4 umkristallisiert.

Die Reaktion kann im Dünnschichtchromatogramm verfolgt werden (Kieselgel, Toluol/Aceton 9:1).
Man erhält hellgelbe Kristalle
Ausbeute: 6,9 g (48,8 %)
Smp.: 213-214°C
UV/Vis (EtOH): λₘₐₓ (ε) = 350 (31662) nm

| Elementaranalyse: | | | |
|---|---|---|---|
| | %C | %H | %N |
| Berechnet: | 70,06 | 8,37 | 7,43 |
| Gefunden: | 70,4 | 8,6 | 7,5 |

### * Beispiel 6a: Herstellung der Verbindung der Formel (106a):

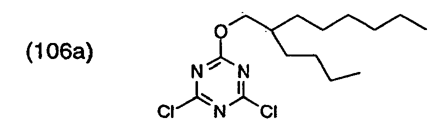

Die Verbindung der Formel (106a) wird analog der Verbindung der Formel (101a) hergestellt. Als Alkoholkomponente wird 2-Butyl-1-octanol verwendet.

### * Beispiel 6b: Herstellung der Verbindung der Formel (106):

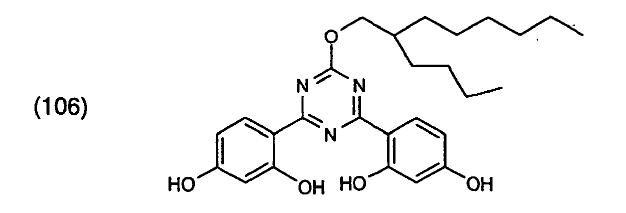

Die Verbindung der Formel (106a) (16,7 g, 0,05 mol), Toluol (150 ml) und Resorcin (13,2 g, 0,12 mol) werden bei Raumtemperatur vorgelegt. Bei 0-5°C wird innerhalb von 1h 15min. pulverisiertes Aluminiumchlorid (14,7 g, 0,11 mol) in kleinen Portionen eingetragen und 6,5 Stunden bei 0-5°C nachgerührt. Dann wird 2N-HCI (60 ml) unter Kühlung in das Reaktionsgemisch eingetropft. Die gelbe Emulsion wird mit Butylmethylether extrahiert. Die organische Phase wird mit 10%iger NaCl-Lösung gewaschen, über Na₂SO₄ getrocknet und vom Lösungsmittel befreit. Der feste Rückstand wird säulenchromatographisch gereinigt (Kieselgel, Toluol/Butylmethylether 75:25).
Die Reaktion kann im Dünnschichtchromatogramm verfolgt werden (Kieselgel, Toluol/Aceton 9:1).
Man erhält beige Kristalle
Ausbeute: 7,0 g (29,1 %)
Smp.: 170-172°C
¹³C NMR (90MHz, D₆2DMSO, TMS): δ = 14,7; 23,0; 23,3; 27,0; 29,3; 29,9; 31,2; 31,5; 32,1; 37,5; 67,2; 103,9; 109,2; 109,5; 131,6; 164,5 (C_{q}); 165,1 (C_{q}); 168,0 (C_{q}); 171,6 (C_{q}).

| Elementaranalyse: | | | |
|---|---|---|---|
| | %C | %H | %N |
| Berechnet: | 67,34 | 7,33 | 8,73 |
| Gefunden: | 67,3 | 7,4 | 8,5 |

### * Beispiel 7a: Herstellung der Verbindung der Formel (107a):

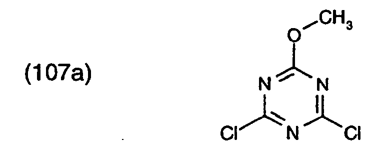

Die Verbindung der Formel (107a) wird analog der Verbindung der Formel (101a) hergestellt. Als Alkoholkomponente wird Methanol verwendet.

### *Beispiel 7b: Herstellung der Verbindung der Formel (107)

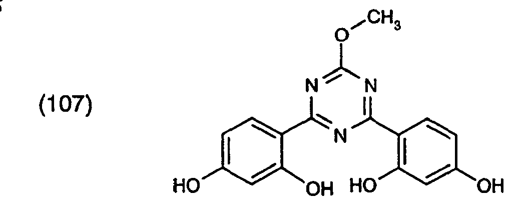

Die Verbindung der Formel (107) wird analog der Verbindung der Formel (105) hergestellt. Das Rohprodukt wird in warmem Methanol (150 ml) verrührt, abgenutscht, mit Methanol gewaschen, getrocknet und aus Acetonitril/N-Methyl-2-pyrrolidon umkristallisiert. Das so erhaltene Kristallpulver wird mit Methanol ausgekocht, um eingeschlossenes N-Methyl-2-pyrrolidon zu entfernen, abfiltriert und getrocknet.
Die Reaktion kann im Dünnschichtchromatogramm verfolgt werden (Kieselgel, Toluol/Aceton 8:2).
Man erhält hellgelbe Kristalle.

Die Verbindung ist in der EP-A-0,165,608 beschrieben.
Löslichkeit in Ethanol (25°C): 0,08%
Smp.: >300°C
UV/Vis (EtOH): λₘₐₓ (ε) = 350 (36949) nm
¹³C NMR (90MHz, D₆-DMSO, TMS): δ = 53,9 (OCH₃); 102,0(CH); 107,6(CH);; 130,0(CH);; 107,3 (C_{q}); 162,5 (C_{q}); 163,2 (C_{q}); 166,2 (C_{q}); 169,5 (C_{q}).

| Elementaranalyse: | | | |
|---|---|---|---|
| | %C | %H | %N |
| Berechnet: | 58,72 | 4,00 | 12,84 |
| Gefunden: | 58,64 | 4,18 | 12,76 |

### * Beispiel 8a: Herstellung der Verbindung der Formel (108a):

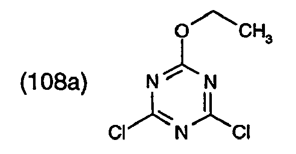

Die Verbindung der Formel (108a) wird analog der Verbindung der Formel (101a) hergestellt. Als Alkoholkomponente wird Ethanol verwendet.

### *Beispiel 8b: Herstellung der Verbindung der Formel (108):

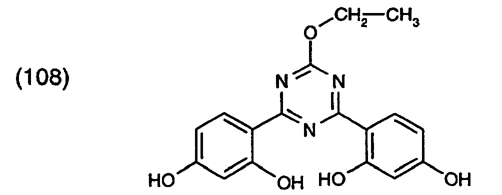

Die Verbindung der Formel (108) wird analog der Verbindung der Formel (105) hergestellt. Die Reaktion kann im Dünnschichtchromatogramm verfolgt werden (Kieselgel, Toluol/Aceton 8:2).
Man erhält hellbeige Kristalle.
Löslichkeit in Ethanol (25°C): 0,17%
Ausbeute: 3,6 g (42,2 %)
Smp.: >300°C
UV/Vis (EtOH): λₘₐₓ (ε) = 350 (36761) nm
¹³C NMR (90MHz, D₆-DMSO, TMS): δ = 14,8 (CH₃) ;64,9 (CH₂);.103,8 (CH); 109,5 (CH); 131,8 (CH); 109,1 (C_{q}); 164,4 (C_{q}); 165,0 (C_{q}); 167,5(C_{q}); 171,4 (C_{q}).

### *Beispiel 9a: Herstellung der Verbindung der Formel (109a):

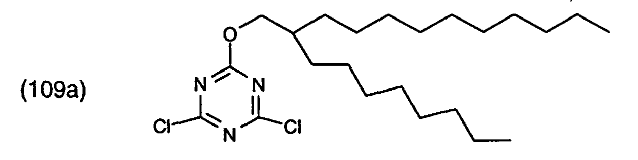

Die Verbindung der Formel (109a) wird analog der Verbindung der Formel (101a) hergestellt. Als Alkoholkomponente wird 2-Octyl-1-Dodecanol verwendet.

### *Beispiel 9b: Herstellung der Verbindung der Formel (109):

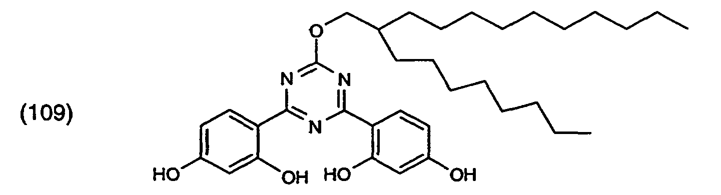

Resorcin (13,2 g, 0,12 mol) wird in Nitrobenzol (30 ml) vorgelegt. Bei 50-60°C wird eine Lösung der Verbindung der Formel (109a) (22,3 g, 0,05 mol) in Toluol (70 ml) zugegeben. Bei 0-5 °C wird innerhalb von 40 min pulverisiertes Aluminiumchlorid (14,7 g, 0,11 mol) eingetragen und 6 Stunden bei 0-5°C nachgerührt. Anschliessend wird das Reaktionsgemisch in 2N HCl eingerührt und das Nitrobenzol durch Wasserdampfdestillation abgezogen. Der feste Rückstand wird abgetrennt und mit Butylmethylether extrahiert. Die organische Phase wird mit 10%iger NaCl-Lösung und 2%iger Na₂CO₃-Lösung gewaschen, über Na₂SO₄ getrocknet und eingedampft. Die Reinigung des Rohprodukts erfolgt säulenchromatographisch (Kieselgel, Toluol/Aceton 85:15). Analysenreines Produkt wird durch nachfolgendes Umkristallisieren aus Aceton erhalten.

Die Reaktion kann im Dünnschichtchromatogramm verfolgt werden (Kieselgel, Toluol/Aceton 9:1).
Man erhält hellgelbe Kristalle.
Ausbeute: 7,1 g (23,9 %)
Smp.: 166-167°C
¹³C NMR (90MHz, D₆-DMSO, TMS): δ = 14,6; 23,0; 27,0; 29,6; 29,7; 29,9; 28,0; 30,2; 30,3; 31,4; 32,2; 37,6; 71,2; 103,9; 109,1; 109,5; 131,8; 164,5 (C_{q}); 165,1 (C_{q}); 168,0 (C_{q}); 171,6 (C_{q}).

| Elementaranalyse: | | | |
|---|---|---|---|
| | %C | %H | %N |
| Berechnet: | 70,79 | 8,66 | 7,08 |
| Gefunden: | 70,8 | 8,5 | 6,9 |

### *Beispiel 10: Herstellung der Verbindung der Formel (110):

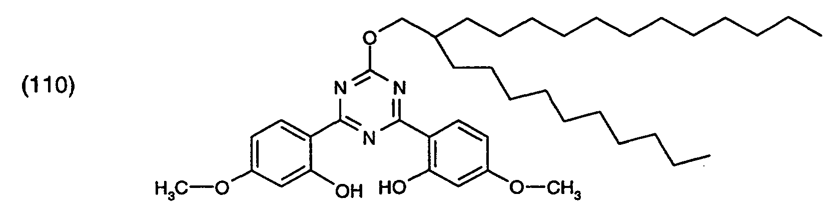

Die Verbindung der Formel (101) (9,8 g, 0,015 mol), Aceton (85 ml), Wasser (40 ml) und 2N NaOH (15,8 ml) werden bei Raumtemperatur vorgelegt. Bei 40°C wird Dimethylsulfat (4,16 g, 0,04 mol) zugetropft und 2,5 Stunden bei 40°C nachgerührt. Mit 1N HCl wird auf pH 6 gestellt und der Feststoff abfiltriert. Der Filterkuchen wird in Toluol gelöst, mit H₂O ausgeschüttelt, getrocknet und vom Lösungsmittel befreit. Anschließend wird säulenchromatographisch (Kieselgel, Toluol) getrennt und aus Diethylether umkristallisiert.
Die Reaktion kann im Dünnschichtchromatogramm verfolgt werden (Kieselgel, Toluol/Aceton 95:5)
Man erhält hellbeige Kristalle.
Ausbeute: 3,1 g (30,5 %)
Smp.: 79-80°C
UV/Vis (Dioxan): λₘₐₓ (ε) = 347 (36219) nm
¹³C NMR (90MHz, CDCl₃. TMS): δ= 15,5; 24,1; 28,2; 30,8; 31,1; 31,4; 32,5; 33,3; 38,9; 56,9 (OCH₃); 72,7; 102,7; 109,4; 111,2 (COCH₃); 132,4; 165,9 (C_{q}); 167,1 (C_{q}); 168,8 (C_{q}); 172,8 (C_{q}).

| Elementaranalyse: | | | |
|---|---|---|---|
| | %C | %H | %N |
| Berechnet: | 72,64 | 9,37 | 6,20 |
| Gefunden: | 72,77 | 9,39 | 6,20 |

### *Beispiel 11: Herstellung der Verbindung der Formel (111);

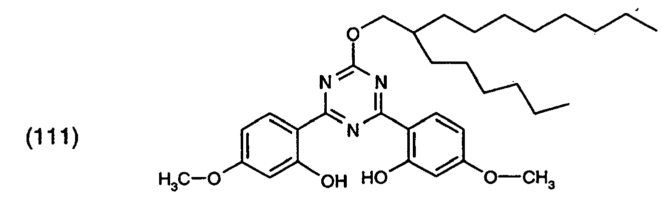

Die Verbindung der Formel (104) (10,8 g, 0,02 mol) wird in Dioxan (100 ml) vorgelegt. Bei 40°C wird 2N NaOH (21 ml) zugegeben und 15 min nachgerührt. Anschließend wird Dimethylsulfat (4,2 ml) bei 40°C zudosiert und 5 Stunden nachgerührt. Das Lösungsmittel wird am Rotationsverdampfer abgezogen und der Rückstand in Toluol gelöst. Die organische Phase wird mit 10%iger NaCl-Lösung ausgeschüttelt und getrocknet. Reines Produkt wird durch säulenchromatographische Trennung (Kieselgel, Toluol/Aceton 98,5:1,5) und anschliessendes Umkristallisierenaus Hexan erhalten.
Die Reaktion kann im Dünnschichtchromatogramm verfolgt werden (Kieselgel, Toluol/Aceton 9:1).
Man erhält hellbeige Kristalle.
Ausbeute: 7,5 g (66,3 %)
Smp.: 99-100°C
UV/Vis (EtOH): λₘₐₓ (ε) = 348 (34123) nm
¹³C NMR (90MHz, CDCl₃, TMS): δ= 14,5; 23,1; 27,20; 27,21; 29,8; 30,0; 30,1; 30,4; 31,5; 32,27; 32,31; 38,9; 55,8 (OCH3); 71,7; 101,6; 108,4; 110,2; 131,2; 164,9 (C_{q}); 166,0 (C_{q}); 167,8 (C_{q}); 172,0 (C_{q}).

| Elementaranalyse: | | | |
|---|---|---|---|
| | %C | %H | %N |
| Berechnet: | 70,06 | 8,37 | 7,43 |
| Gefunden: | 70,0 | 8,4 | 7,1 |

### * Beispiel 12: Herstellung der Verbindung der Formel (112):

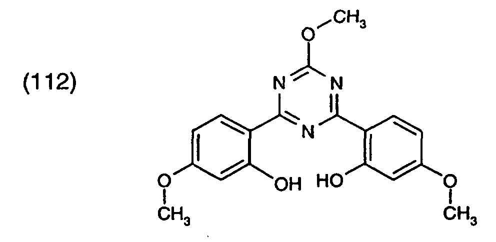

Die Verbindung der Formel (112) wird analog der Verbindung der Formel (110) hergestellt.
Als Lösungsmittel wird Dioxan verwendet. Das Nutschgut wird mit Dioxan/H₂O und Methanol gewaschen und getrocknet. Anschliessend wird aus Methylcellosolve umkristallisiert.
Man erhält hellgelbe Kristalle.
Ausbeute: 5,7 g (64,2 %)
Smp.: 193-194°C
UV/Vis (Dioxan): λₘₐₓ (ε) = 347 (37680) nm
Applikationsbeispiele:

### Beispiel 13: Mikronisierung

50g der Verbindung der Formel (108)* werden zusammen mit 7% Alkylpolyglykosid, 43% H₂O und 80g Zirkoniumsand gemahlen, bis eine Partikelgröße von d₅₀ = 150 nm erreicht ist.
Danach wird der Mahlsand von der Suspension, enthaltend die mikronisierte Verbindung der Formel (108) abgetrennt.

### Beispiel 14: Sonnenschutzformulierung (W/O)

| | Komponenten | Gew.% |
|---|---|---|
| Öl-Phase | PEG-30 Dipolyhydroxystearate (Arlacel P 135^{®}) | 3,00 |
| | PEG-22/ Dodecyl Glycol Copolymer (Elfacos ST 37^{®}) | 1,00 |
| | Microcrystalline Wax | 1,00 |
| | Hydrogeniertes Castor Öl | 0,50 |
| | Magnesium Stearate | 1,00 |
| | Octyl Stearate | 15,00 |
| | Coco Glycerides | 2,00 |
| | Mineral Öl | 3,00 |
| | Phenoxyethanol&Parabens | 1,00 |
| | Octyl Methoxycinnamate | 5,00 |
| | Dimethicone | 0,10 |
| | | |
| Wasser-Phase | deionisiertes Wasser | 52,40 |
| | Magnesium Sulphate (MgSO₄ x 7 H₂O) | 1,00 |
| | Propylene Glycol | 4,00 |
| | | |
| | 50%ige Suspension entsprechend Beispiel 13 | 10.00 |

### Herstellungsvorschrift:

Die Öl- und die Wasserphase werden separat auf 80° C erhitzt, zusammengegeben und die Mischung intensiv homogenisiert. Danach lässt man unter leichtem Rühren auf 40°C abkühlen. Die 50%ige Suspension des mikronisierten UV-Absorbers der Formel (108)* wird portionsweise unter Rühren zugegeben und noch 15 Minuten nachgerührt.
SPF (in vivo) = 16 (COLIPA)
[ohne mikronisierte Verbindung der Formel (108): SPF = 6]

### Beispiel 15: Sonnenschutzemulsion (O/W)

| | Komponenten | Gew.-% |
|---|---|---|
| A | Polyglyceryl-3 Methylglucose Distearate (Tego^{®} Care 450) | 2,0 |
| | Decyl Oleate | 5,7 |
| | Isopropyl Palmitate | 5,0 |
| | Caprylic/Capric Triglyceride | 6,5 |
| | Verbindung der Formel (101)* | 3,5 |
| | | |
| B | Glycerol | 3,0 |
| | Phenonip | 0,5 |
| | deionisiertes Wasserer | 72,4 |
| | | |
| C | Carbomer 141 | 0,2 |
| | Isopropyl Palmitate | 0,8 |
| | | |
| D | NaOH (10%) | 0,4 |

### Herstellungsvorschrift:

Die Phasen A und B werden separat auf 80° C erwärmt und dann unter leichtem Rühren zusammengegeben. C wird zu der Mischung von A und B zugegeben und dannach intensiv homogenisiert. Das Homogenisat läßt man unter leichtem Rühren auf Raumtemperatur abkühlen. Wenn erforderlich wird der pH durch Hinzufügen von D eingestellt.
- SPF (in vitro) = 3,0: (Optometrics SPF 290 Analyzer, 2 µl/cm² on Transpore^{®}-Tape) Australian / New Zealand Standard, 15 / NZS 2604 : 1993 (weniger als 10% Transmission zwischen 320 nm und 360 nm) wird erfüllt.

### Beispiel 16: Sonnenschutzemulsion (O/W)

| | Komponenten | Gew.-% |
|---|---|---|
| A | Polyglyceryl-3 Methylglucose Distearate (Tego^{®} Care 450) | 2,0 |
| | Decyl Oleate | 5,7 |
| | Isopropyl Palmitate | 5,0 |
| | Caprylic/Capric Triglyceride | 6,5 |
| | Verbindung der Formel (101)* | 3,0 |
| | Octyl Methoxycinnamate | 5,0 |
| | | |
| B | Glycerol | 3,0 |
| | Phenonip | 0.5 |
| | deionisiertes Wasser | 67,9 |
| | | |
| C | Carbomer 141 | 0,2 |
| | Isopropyl Palmitate | 0,8 |
| | | |
| D | NaOH (10%) | 0,4 |

### Herstellungsvorschrift:

Die Phasen A und B werden separat auf 80° C erwärmt und dann unter leichtem Rühren zusammengegeben. C wird zu der Mischung von A und B zugegeben und danach intensiv homogenisiert. Das Homogenisat lässt man unter leichtem Rühren auf Raumtemperatur abkühlen. Wenn erforderlich wird der pH durch Hinzufügen von D eingestellt.
- SPF (in vitro) = 11: (Optometrics SPF 290 Analyzer, 2 µl/cm² on Transpore^{®}-Tape) Australian / New Zealand Standard, 15 / NZS 2604 : 1993 (weniger als 10% Transmission zwischen 320 nm und 360 nm) wird erfüllt.

### Beispiel 17: Sonnenschutzcreme (W/O)

| | Komponenten | Gew.-% |
|---|---|---|
| Ölphase | Methoxy PEG-22/Dodecyl Glycol Copolymer (Elfacos E 200^{®}) | 3,00 |
| | PEG-22/ Dodecyl Glycol Copolymer (Elfacos ST 37^{®}) | 3,00 |
| | Hydroxyoctacosanyl Hydroxystearate (Elfacos C 26^{®}) | 3,00 |
| | Octyl Stearate | 15,00 |
| | Coco Glycerides | 2,00 |
| | Mineral Öl | 3,00 |
| | Phenoxyethanol&Parabens | 0,70 |
| | Verbindung der Formel (104) | 3,50 |
| | 4-Methyl-benzylidene Camphor | 4,00 |
| | Tocopheryl Acetate | 1,00 |
| | Dimethicone | 0,20 |
| | | |
| Wasser-Phase | deionisiertes Wasser | 56,80 |
| | Magnesium Sulphate (MgSO₄ x 7 H₂O) | 0,80 |
| | Propylene Glycol | 4,00 |

### Herstellungsvorschrift:

Die Phasen A und B werden separat auf 80° C erwärmt und dann unter leichtem Rühren zusammengegeben. C wird zu der Mischung von A und B zugegeben und danach intensiv homogenisiert. Das Homogenisat lässt man unter leichtem Rühren auf Raumtemperatur abkühlen. Wenn erforderlich wird der pH durch Hinzufügen von D eingestellt.
- SPF (in vitro) = 12: (Optometrics SPF 290 Analyzer, 2 µl/cm² on Transpore^{®}-Tape) Australian / New Zealand Standard, 15 / NZS 2604 : 1993 (weniger als 10% Transmission zwischen 320 nm und 360 nm) wird erfüllt.

### Beispiel 18: Sonnenschutzcreme

| | Komponenten | Gew.-% |
|---|---|---|
| 1 | deionisiertes Wasser | ad 100 |
| 2 | Titanium Dioxide (and) Isopropyl Myristate | 6,25 |
| 3 | Phenoxyethanol&Parabens | 0,50 |
| 4 | Salcare SC91 | 2,50 |
| 5 | Glycerin | 2,00 |
| | | |
| 6 | Verbindung der Formel (104) | 0,70 |
| 7 | Isopropyl Palmitate | 5,00 |
| 8 | Caprylic/Capric Triglyceride | 2,50 |
| | | |
| 9 | Propylene Glycol | 1,00 |
| | | |
| 10 | Methylen Bis Benzotriazolyl Tetramethyl-butylphenol (50%ige Suspension) | 6,00 |

### Herstellungsvorschrift:

Die Substanzen (2) bis (5) werden in der angegebenen Reihenfolge unter kräftigem Rühren zu Wasser (1) zusammengegeben. Danach wird die Lösung von (6) im Gemisch von (7) und (8) unter mässigem Rühren zugegeben. Es folgen ebenfalls unter Rühren die Zugabe von (9) und danach von (10). Man rührt weiter, bis die Masse homogen ist.

Australian/New Zealand Standard, 15/NZS 2604:1993 (weniger als 10% Transmission zwischen 320 nm und 360 nm) wird erfüllt.

## Patentansprüche

1. Verbindungen der Formel 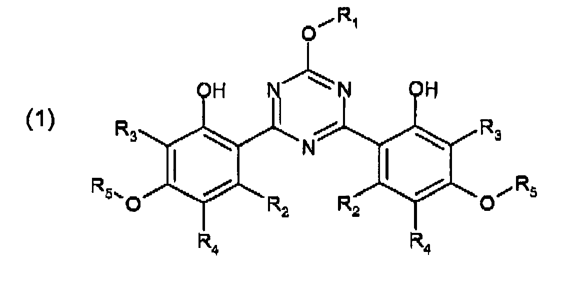 worin
R₁ 2-Decyl-hexadecyl; isooctadecyl; n-octadecyl; 2-hexyldecyl; 2-ethylhexyl;
R₂, R₃ und R₄,unabhängig voneinander Wasserstoff, Hydroxy-, C₁-C₃₀-Alkyl, C₁-C₃₀-Alkenyl, und
R₅ Wasserstoff;
bedeuten,

2. Verfahren zur Herstellung der Verbindungen der Formel (1) gemäss Anspruch 1, **dadurch gekennzeichnet, dass** man in der ersten Reaktionsstufe Cyanurchlorid mit der Verbindung R₁-OH zu den Dichlor-aryloxytriazinen der Formel (2) umsetzt, und in einer zweiten Stufe in einer Friedel-Crafts-Reatkion diese Verbindungen mit Resorcin zur Verbindung der Formel (1) umsetzt nach folgendem Reaktionsschema:

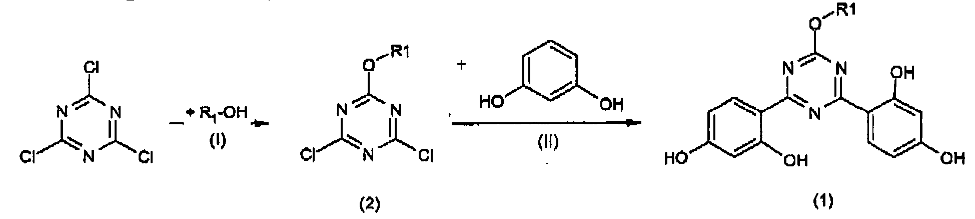

3. Kosmetisches Präparat, enthaltend mindestens eine oder mehrere Verbindungen der Formel (1) nach Anspruch 1 mit kosmetisch verträglichen Träger- oder Hilfsstoffen.

4. Präparat nach Anspruch 3, **dadurch gekennzeichnet, dass** es weitere UV-Schutzstoffe enthält,

5. Präparat nach Anspruch 3 oder 4, **dadurch gekennzeichnet, dass** es als weitere UV- Schutzstoffe Triazine. Oxanilide, Triazole, Vinylgruppen enthaltende Amide oder Zimtsäureamide enthält.

6. Zusammensetzung enthaltend
(A) ein gegen Schädigung durch Licht, Sauerstoff und/oder Hitze empfindliches organisches Material und
(B) als Stabilisator eine Verbindung gemäss Anspruch 1.

## Claims

1. A compound of the formula 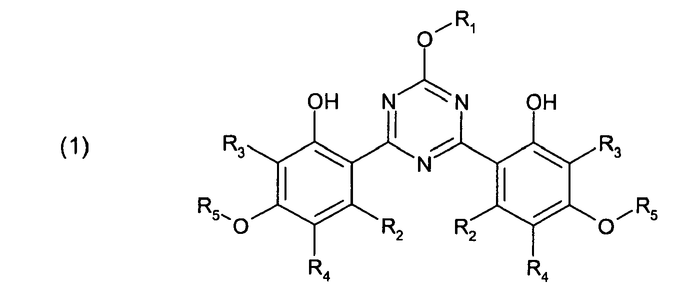 in which
R₁ is 2-decylhexadecyl; isooctadecyl; n-octadecyl; 2-hexyldecyl; 2-ethylhexyl;
R₂, R₃ and R₄, independently of one another, are hydrogen, hydroxyl, C₁-C₃₀alkyl, C₁-C₃₀alkenyl, and
R₅ is hydrogen.

2. A process for the preparation of the compounds of the formula (1) according to claim 1, wherein, in the first reaction stage, cyanuric chloride is reacted with the compound R₁-OH to give the dichloroaryloxytriazines of the formula (2) and, in a second stage, in a Friedel-Crafts reaction, these compounds are reacted with resorcinol to give the compound of the formula (1), in accordance with the following reaction equation:

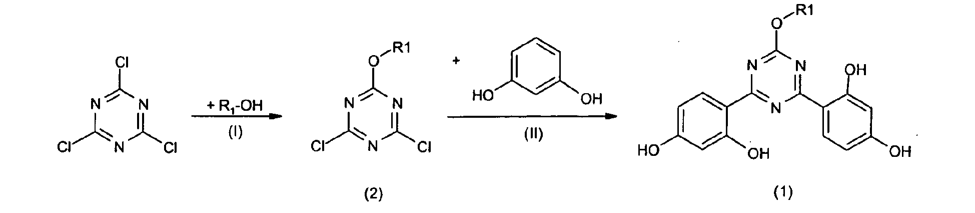

3. A cosmetic preparation comprising at least one or more compounds of the formula (1) according to claim 1 with cosmetically compatible carriers or auxiliaries.

4. A preparation according to claim 3, which comprises further UV protective substances.

5. A preparation according to claim 3 or 4, which comprises, as further UV protective substances, triazines, oxanilides, triazoles, amides containing vinyl groups, or cinnamides.

6. A composition comprising
(A) an organic material which is sensitive to damage by light, oxygen and/or heat, and
(B) as stabilizer, a compound according to claims 1.

## Revendications

1. Composés de formule : 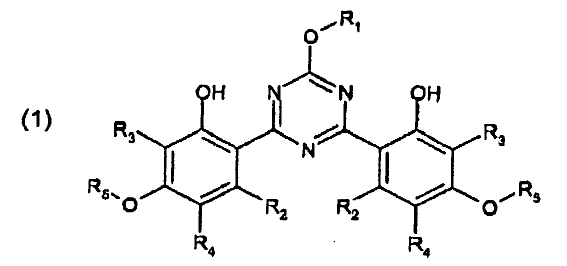 dans laquelle,
R₁ est un radical 2-décyl-hexadécyl, isooctadécyl, n-octadécyl, 2-hexyldécyl ou 2-éthylhexyl;
R₂, R₃ et R₄, sont, indépendamment l'un de l'autre, un atome d'hydrogène, un groupe hydroxy-, un radical alkyl en (C₁-C₃₀) ou un radical alkényl en (C₁-C₃₀), et
R₅ est un atome d'hydrogène;

2. Procédé de fabrication de composés de formule (1) selon la revendication 1,
**caractérisé en ce que**, dans une première étape réactionnelle, on prépare une dichloro-aryloxytriazine de formule (2) par réaction du composé R₁-OH avec du chlorure de cyanure et que, dans une seconde étape, on soumet ce dernier composé à une réaction de Friedel-Crafts en présence de résorcine pour obtenir le composé de formule (1) selon le schéma réactionnel suivant:

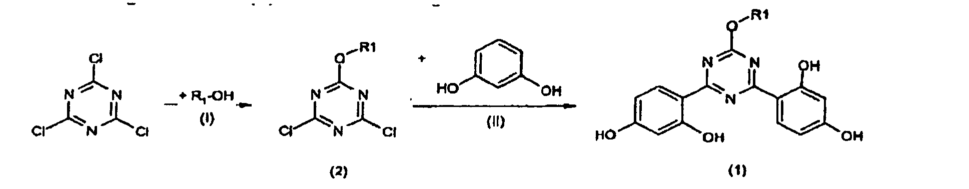

3. Préparation cosmétique contenant au moins un ou plusieurs composés de formule (1) et un support ou adjuvant acceptable du point de vue cosmétologique.

4. Préparation selon la revendication 3, **caractérisée en ce qu'**elle contient des substances supplémentaires consistant en des produits de protection à l'égard des UV.

5. Préparation selon la revendication 3 ou 4, **caractérisée en ce que**, en tant que substances supplémentaires, elle contient des produits de protection à l'égard des UV consistant en la triazine, l'oxanilide, le triazole, les amides contenant des groupes vinyl ou amides de l'acide cinnamique.

6. Composition contenant:
(A) un matériau organique pour la protection contre les atteintes dues à la lumière, aux acides et/ou la chaleur et
(B) en tant que stabilisant un composé selon la revendication 1.
